# EUROPEAN PATENT SPECIFICATION

(11) **EP 1 940 978 B1**
(45) Date of publication and mention of the grant of the patent: **05.01.2011**
(21) Application number: 06807464.0
(22) Date of filing: 23.10.2006
(51) Int. Cl.: C09D 7/00, B01F 17/00, A61K 8/87, A61K 8/33, C11D 1/74, C11D 3/37

(54) **LIQUID THICKENERS FOR AQUEOUS SYSTEMS**
FLÜSSIGE EINDICKER FÜR WÄSSRIGE SYSTEME
EPAISSISSANTS LIQUIDES POUR SYSTEMES AQUEUX

(30) Priority: 27.10.2005 IT VA20050058
(43) Date of publication of application: 09.07.2008
(73) Proprietor: Lamberti Spa, 21041 Albizzate (IT)
(72) Inventor: DI COSMO, Anna, I-00049 Velletri (RM) (IT)
(86) International application number: PCT/EP2006/067654
(87) International publication number: WO 2007/048766

(56) References cited:
- EP-A- 1 403 324
- WO-A-00/00539
- US-A- 4 636 326
- US-A- 5 425 806
- US-A1- 2005 112 081

## Description

### Technical field

The present invention is directed to liquid thickeners for aqueous systems containing polyurethane associative thickeners and ethoxylated glycerides and to their use in the preparation of thickened aqueous systems, such as cosmetics, latex paints, water-based inks and printing pastes, paper coatings agents, detergents.

The liquid thickeners of the invention, advantageously, contain neither an organic solvent, nor volatile organic compounds, nor other viscosity suppressing agents, they can be easily incorporated into aqueous systems and are acceptable for cosmetic use.

### Background art

Polyurethane associative thickeners are well known and described in many publications (such as in US 4,079,028 and US 4,155,892, both assigned to Rohm and Haas).

They are characterised by the simultaneous presence in their structure of urethane groups and hydrophilic and hydrophobic chains.

Their use as thickeners in water based systems, such as latex paints, is widespread.

Nonetheless, they suffer of some disadvantages.

Their incorporation in dry form into aqueous systems is difficult and can involve extensive manipulation, as their hydrophobic chains hinder rapid dissolution in water; on the other hand, their thickening capacity prevents the preparation of highly concentrated aqueous solutions that could be used as liquid thickeners.

Many attempts to prepare highly concentrated thickener compositions containing associative polyurethanes are reported in the literature. It has been suggested: to add solvents and/or surfactants; to reduce the molecular weight of the polyurethane; to incorporate in the thickener formulation specific compounds acting as viscosity reducers (see e.g. US 5,378,756, US 5,425,806, US 5,959,013, US 6,090,876, US 6,150,445). US 5,425,806 and US 6,150,445 disclose liquid aqueous thickeners containing polyurethane associative thickeners, water and a surfactant as the viscosity reducing agent. US 4,636,326 discloses an aqueous fluid composition comprising a polyurethane thickener and a water-soluble polyester that may contain nonionic or anionic surfactants; the surfactants are said to aid the dispersal of the functional additives in the aqueous system.

While performing their intended function, organic solvents often constitute a potential environmental, safety and health hazard.

Certain surfactants, such as alkoxylated phenols, are toxicologically objectionable; others are not so efficient in reducing the viscosity of the thickener, especially in the case of highly active, extremely pseudoplastic thickeners, or they give rise to unstable compositions, or they adversely affect some properties in applications.

The environmental and toxicity hazard related to the use of solvents and certain surfactants as well as the unsatisfactory efficiency of the thickener of prior art has prompted the applicant to perform an extensive research with the scope of providing a liquid thickener, based on associative polyurethanes, which is pourable and can be easily incorporated into aqueous systems, is effective, is stable over time, is acceptable for cosmetic use, and is devoid of organic solvents and volatile organic compounds.

### Disclosure of Invention

It has now been found that a liquid thickener for aqueous systems containing, as viscosity suppressing agent, from 5 to 40% wt of ethoxylated mono- and/or di-glycerides of carboxylic acids having from 6 to 18 carbon atoms, from 15 to 40% wt of a polyurethane associative thickener, obtained from the reaction of at least one organic polyisocyanate, at least one water soluble polyether diol and at least one hydrophobic capping agent containing from 10 to 24 carbon atoms, from 20 to 80% wt of water, and which is devoid of organic solvents, volatile organic compounds and other viscosity suppressing agents, exhibits the characteristics mentioned in the above paragraph.

Surprisingly, it has been found that ethoxylated mono- and/or di-glycerides of carboxylic acids having from 6 to 18 carbon atoms, while being more effective as viscosity reducers than conventional surfactants, are able to generate liquid thickeners based on associative polyurethanes which exhibit a very good thickening efficiency, and also have a very favourable toxicological profile.

According to a particularly advantageous aspect of the invention, the above described ethoxylated mono and/or di-glycerides are also effective as stabiliser of the liquid thickeners.

The mono and/or di-glycerides useful for the realisation of the present invention are known and described for example in US 3,934,003. They can be prepared, for example, by reacting ethylene oxide with partial glycerides, which can be obtained by fractionating natural oil fatty acids, or by ethoxylation of glycerine followed by esterification of the resulting product with fatty acids.

They are commonly used as emulsifiers in cosmetics and pharmaceuticals, lubricants, mold release compounds, plasticizers...

In cosmetics, ethoxylated mono- and/or di-glyceuides of fatty acids are particularly appreciated, as they also act as emollient components.

The mono and/or di-glycerides of the invention are water soluble, and are ethoxylated with from 4 to 12 moles of ethylene oxide.

According to a preferred aspect of the invention, the liquid thickener comprises an associative polyurethane obtained from the reaction of at least one organic polyisocyanate, at least one water soluble polyether diol and at least one hydrophobic capping agent containing from 12 to 24 carbon atoms.

Examples of utilisable polyurethanes are linear or branched polyurethanes obtained from the reaction of polyethylene glycol or polypropylene glycol (having an average molecular weight of 4,000 to 14,000), linear or branched monohydric alcohols containing from 10 to 24 carbon atoms and of one ore more isocyanates selected from hexamethylene-1,6-diisocyanate, 1-isocyanatomethyl-5-isocyanato-1,3,3-trimethylcyclohexane, 4,4'-dicyclohexyl methane diisocyanate, 1,6-diisocyanato-2,4,4-trimethylhexane, 2,6- and 2,4-tolylenediisocyanate, hexamethylene-1,6-diisocyanate isocyanurate, optionally in the presence of a polyfunctional alcohol (such as trimethylolpropane); the polyurethanes generally have an average molecular weight of 6,000 to 30,000.

The liquid thickeners of the invention are devoid of solvents, volatile organic compounds and other viscosity suppressing agents, i.e. they contain less than 1% wt of the sum of said substances.

The associative polyurethane contained in the liquid thickener of the invention may be newtonian, i.e. highly effective at mid-high shear rate, or even pseudoplastic, i.e. highly effective at low shear rate.

It has been found that ethoxylated mono- and/or di-glycerides of saturated fatty acids having from 8 to 14 carbon atoms, ethoxylated with 6 to 7 moles of ethylene oxide, are particularly suitable as viscosity reducers even in the case of highly active, extremely pseudoplastic polyurethanes.

In the present text, with the expression "extremely pseudoplastic polyurethane" we mean an associative polyurethane thickener prepared from hydrophobic capping agents containing at least 16 carbon atoms.

Liquid thickeners comprising associative compounds are normally used to thicken binder-containing compositions, such as water-based paper coating agents and water based varnish and paints.

Suitable binders are emulsion binders, such as alkyd resins, and latex binders, such as polyvinyl acetate, copolymers of vinyl acetate and acrylate, and/or ethylene, and/or vinyl chloride, and/or styrene, polyacrylates.

The liquid thickeners of the invention have proven to be suited to thicken aqueous based binder-containing compositions.

A particularly preferred liquid thickener according to the present invention, which, unexpectedly, has been found well suited for use even in binder-free aqueous compositions, contains from 5 to 30% wt of mono and/or di-glycerides of caprylic and capric acids ethoxylated with about 6 moles of ethylene oxide, from 15 to 30% wt of an associative polyurethane obtained from the reaction of polyethylene glycol, 4,4'-dicyclohexyl methane diisocyanate and/or 1-isocyanatomethyl-5-isocyanato-1,3,3-trimethylcyclohexane and C ₁₆ -C₂₄ branched monohydric alcohol, preferably C ₁₆ or C₂₀ Guerbet alcohol, and having a molecular weight of about 6,000 to 20,000, and from 40 to 80% wt of water; this preferred liquid thickener is colourless, transparent, remarkably stable over time and can be advantageously used to obtain thickened, transparent and colourless cosmetic and detergent formulations, even in the absence of an organic binder.

The liquid thickeners of the invention can be prepared by reacting at least one organic polyisocyanate, at least one water soluble polyether diol and at least one hydrophobic capping agent to obtain an associative polyurethane, and then mixing the polyurethane with the proper amount of ethoxylated mono- and/or di-glycerides of carboxylic acids having 6 to 18 carbon atoms and adjusting the amount of water prior or after the mixing step.

The associative polyurethanes are prepared according to techniques generally known for the synthesis of urethanes, preferably in such a way that no isocyanate remains unreacted.

Water should be excluded from the reaction since it would react with the isocyanate functionality.

Anhydrous conditions are achieved by preliminary drying of reactants.

The reaction is run preferably without any solvent.

The reaction temperature should be selected in order to obtain reasonably fast reaction and polymer viscosity while avoiding undesirable side reaction.

According to a preferred embodiment, the liquid thickener according to the invention is prepared by adding the ethoxylated mono and/or di-glyceride immediately after the preparation of the associative polyurethane, while still in molten form.

If the above described melt or solvent free process is used to prepare the associative polyurethane, the polyglycol is placed in a mixer, heated and de-watered for several hours.

The vacuum is released and the temperature is stabilised.

The polyisocyanate and catalyst are added and after a suitable reaction time, the capping agent is added and allowed to react. The ethoxylated mono and/or di-glyceride is then preferably added to the molten polyurethane and then the polyurethane is dispersed in water.

Alternatively, the liquid thickener of the invention can be obtained by mixing the proper amounts of associative polyurethane in dry form, of ethoxylated mono- and/or di-glycerides of carboxylic acids having from 6 to 18 carbon atoms and the proper amount of water.

It is another object of the present invention a process for the preparation of liquid thickeners, comprising the following steps: a) reacting at least an organic polyisocyanate, at least a water soluble polyether diol and at least a hydrophobic capping agent containing from 10 to 24 carbon atoms, to obtain an associative polyurethane, preferably in molten form; b) mixing the polyurethane, preferably in molten form, with from 0.1 to 2.7 parts by weight of ethoxylated mono and/or di-glycerides of carboxylic acids having from 6 to 18 carbon atoms and with from 0.5 to 5.3 parts by weight of water.

The liquid thickener according to the invention are particularly suited for the preparation of cosmetics, such as shampoos, hair lotions and creams, masks; latex paints; water-based paper coatings; cleaners, in particular household cleaners containing acids as the active ingredients, such as metal cleaners, de-scalant, toilet bowl cleaners, automatic dishwasher rinse additives, and the like.

The liquid thickeners of the invention are characterised by an exceptionally low viscosity combined with high thickening efficiency, even when they contain highly active associative polyurethanes.

Moreover, the ethoxylated glycerides of the invention permit to obtain stable aqueous compositions that do not separate during stock at room temperature and are easy to handle during synthesis of the thickener.

EXAMPLES

**Example 1-6. Viscosity of liquid thickeners prepared from a molten polyurethane.**

Example 1

A reaction vessel, equipped with internal thermometer, stirrer and cooler, is filled, under nitrogen atmosphere and at room temperature with 254.3 g of a 4,000 average molecular weight polyethyleneglycol.

The polyethyleneglycol is melted, heated to 110 °C and dehydrated for 3 hours under vacuum; the temperature is then stabilised at 75 °C and the reactor content placed under a nitrogen blanket.

28.3 g of 1-isocyanatomethyl-5-isocyanato-1,3,3-trimethykyclohexane (isophoronediisocyanate or IPDI) are then added to the reactor together with 2.8 g of trimethylolpropane and 0.1 g of dibutyltin dilaurate as catalyst, under stirring.

The temperature is raised to 110 °C and the reaction is allowed to proceed until the titrimetric determination of the free NCO-groups gives the theoretical value (value determined in this example as well as in the other examples according to the standard method ASTM D2572).

Subsequently, 6.8 g of Isalchem 123 (C ₁₂-C₁₃ primary monobranched alcohols from Condea) and 7.7 g of Isalchem 145 (C ₁₄-C₁₅ primary monobranched alcohols produced by Condea) are added.

After 1 hour of reaction, the polymer is checked to be NCO-negative according to the I.R. spectrum.

Finally, the molten polymer is diluted with 300 g of Sterol CC 595 (PEG-6 capric-caprylic glyceride, from Cesalpinia Chemicals) and 400 g of demineralized water to give a solvent free liquid thickener made of about 30% polyurethane/30% Sterol CC 595 / 40% water, having Brookfield Viscosity at 20 °C of 7,320 mPa*s (S06; 50 rpm).

The obtained liquid thickener is transparent and colourless.

Examples 2-6 (comparative examples).

The procedure of Example 1 was repeated with the exception that Sterol CC 595 was replaced by the nonionic viscosity suppressing agents listed below, to obtain liquid thickeners having the composition and Brookfield viscosity at 20 °C reported in Table 1.

Viscosity suppressing agents (trade name=chemical mature, producer):

Rolfor 123/6.5 = C₁₂-C₁₃ alcohol 6.5 OE, Cesalpinia Chemicals;

Rolfor TR/40 = Isotridecyl Alcohol 40 OE, Cesalpinia Chemicals;

Rolfor TR8/L = Isotridecyl Alcohol 8 OE, Cesalpinia Chemicals;

Borchigen DFN = Benzyl p-hydroxy biphenyl ethoxylated, Borchers.

The viscosity data reported in Table 1 show that the ethoxylated glyceride of the invention lowers the viscosity of the hydrophobically modified polyurethane thickener better than commercially available viscosity suppressing agents, even at high percentage of active matter (polyurethane).

**Table 1**

| Example | Viscosity | Polyurethane | Viscosity | Viscosity |
|---|---|---|---|---|
| | suppressing agent | % (w/w) | suppressing agent % (w/w) | (mPa*s) |
| 1 | Sterol CC595 | 30% | 30% | 7,320 |
| 2* | Rolfor | 30% | 30% | 17,000 |
| | 123/6.5 | | | |
| 3* | Rolfor | 20% | 20% | 9,860 |
| | 123/6.5 | | | |
| 4* | Rolfor TR/40 | 20% | 20% | >100,000 |
| 5* | Rolfor TR8/L | 30% | 30% | >100,000 |
| 6* | Borchigen | 20% | 20% | >100,000 |
| | DFN | | | |

*Comparative examples

**Examples 7-14. Viscosity of liquid thickeners prepared from polyurethane in dry form.**

Preparation of an associative polyurethane (Polyurethane A) in dry form:

A reaction vessel, equipped with internal thermometer, stirrer and cooler, is filled, under nitrogen atmosphere and at room temperature with 255.1 g of a 4,000 average molecular weight polyethyleneglycol. The polyethyleneglycol is melted, heated to 110 °C and dehydrated for 3 hours under vacuum; the temperature is then stabilised at 75 °C and the reactor content placed under a nitrogen blanket.

28.3 g of isophoronediisocyanate are then added to the reactor together with 2.9 g of trimethylolpropane and 0.1 g of dibutyltin dilaurate as catalyst under stirring.

The temperature is raised to 110 °C and the reaction is allowed to proceed until the titrimetric determination of the free NCO-groups gives the theoretical value.

Subsequently 13.6 g of Isalchem 123 are added. After 1 hour of reaction, the polymer is checked to be NCO-negative according to the I.R. spectrum.

The associative polyurethane (Polyurethane A) was obtained as a solid, easily friable wax after cooling.

Preparation of the liquid thickeners:

25 g of Polyurethane A were added to a mixture of 25 g of viscosity suppressing agent (see Table 2) and 50 g of water under vigorous stirring for one hour and then the liquid thickener was stored at 20°C.

Beside those cited above, the viscosity-suppressing agents listed here below were also evaluated:

Viscosity suppressing agents (trade name=chemical nature, producer):

Sterol LG 492 = PEG-7 glyceryl cocoate, Cesalpinia Chemicals;

Rolfor LA 8 = Lauryl alcohol 8 OE, Cesalpinia Chemicals;

Emulson AG 7717/A = Poly-aryl phenol ethoxylated, Cesalpinia Chemicals;

Sorbilene LH = Polyoxyethylene(20) Sorbitan monolaurate, Cesalpinia Chemicals;

Tegodisperse 650 = Modified Polyether , Tego.

The relevant data of the thus obtained liquid thickeners are reported in Table 2.

The viscosity was measured by means of a Brookfield viscometer RVT-DV-I after that each product had lost foam at 20°C.

Stability, expressed in months, is evaluated by visually checking the homogeneity of the sample.

Appearance is also evaluated visually.

The persistence of foam, expressed in days, is also reported in Table 2.

The results show that the ethoxylated glycerides of the present invention are efficient viscosity reducers for polyurethane associative thickeners at high-mid shear rate, such as Polyurethane A.

**Table 2**

| Ex. | Viscosity suppressing agent | Stability | Foam persistence | Appearance | Viscosity (mPa*s) |
|---|---|---|---|---|---|
| 7 | Sterol CC 595 | >6 | 1 | transparent colourless almost | 2,016 |
| 8 | Sterol LG 492 | >6 | <2 | transparent colourless | 4,184 |
| 9* | Borchigen DFN | >6 | <2 | turbid yellowish | 5,304 |
| 10* | Rolfor LA 8 | >6 | 3 | turbid colourless | 5,416 |
| 11* | Emulson AG 7717/A | >6 | 2 | turbid yellowish | 10,140 |
| 12* | Sorbilene LH | >6 | <3 | turbid yellowish | 14,800 |
| 13* | Rolfor 123/6,5 | <1** | >20 | turbid colourless | - |
| 14* | Tegodisperse 650 | <1** | >20 | turbid yellowish | |

*Comparative example

**Separated in two layers

**Examples 15-20. Thickening efficiency of the liquid thickeners.**

Example 15

0.3 g of Polyurethane A were added to 99.7 g of Rolflex D 148, a commercial binder polyurethanic dispersion, from Lamberti SpA.

Example 16-20

1.2 g of liquid thickener of, respectively, Example from 7 to 11 were added to 98.8 g of Rolflex D148.

The active content of associative polyurethane thickener in each mixture of Examples 15-20 is 0.3% by weight.

The thus prepared mixtures were each stirred for five minutes at 600 rpm, and then stored at 20 °C for 24 hours.

The viscosity curves were then measured with a Physica UDS200 rheometer at 20 °C.

The results are summarised in Table 3, where the low shear viscosity is reported.

The viscosity data show that the ethoxylated glycerides do not adversely affect the thickening efficiency of the polyurethane to any great extent.

**Table 3**

| Example | Polyurethane | Viscosity (mPas) (0.1 s-1) |
|---|---|---|
| 15* | Polyurethane A | 18,900 |
| 16 | from Ex. 7 | 15,040 |
| 17 | from Ex. 8 | 12,400 |
| 18* | from Ex. 9 | 16,100 |
| 19* | from Ex. 10 | 12,400 |
| 20* | from Ex. 11 | 12,700 |

*Compartive example

**Examples 21. Viscosity of a liquid thickener prepared from a molten extremely pseudoplastic associative polyurethane.**

A reaction vessel, equipped with internal thermometer, stiller and cooler, is filled, under nitrogen atmosphere and at room temperature with 221.4 g of a 4,000 average molecular weight polyethyleneglycol. The polyethyleneglycol is melted, heated to 110 °C and dehydrated for 3 hours under vacuum; the temperature is then stabilised at 75 °C and the reactor content placed under a nitrogen blanket.

16.4 g of isophoronediisocyanate and 0.1 g of dibutyltin dilaurate as catalyst, are then added to the reactor under stirring. The temperature is raised to 110 °C and the reaction is allowed to proceed until the titrimetric determination of the free NCO-groups gives the theoretical value.

Subsequently 12.1 g of 2-octyldodecanol are added. After 1 hour of reaction, the polymer is checked to be NCO-negative according to the I.R. spectrum. Finally, the molten polymer is diluted with 250 g of Sterol CC 595 and 500 g of demineralized water to give a colourless, transparent liquid thickener made of 25% polymer/25 % Sterol CC595 / 50% water, and having Brookfield viscosity of 4,070 mPa*s (S05; 50 rpm) at 20 °C.

**Examples 22-27. Viscosity of liquid thickeners prepared from an extremely pseudoplastic associative polyurethane in dry form.**

Preparation of the polyurethane (Polyurethane B):

Polyurethane B was prepared as described in Example 21, except that neither Sterol CC 595 nor water were added and the polyurethane was isolated as a solid, easily friable wax.

Preparation of the liquid thickeners:

25 g of Polyurethane B were added to a mixture of 25 g of viscosity suppressing agent (see Table 4) and 50 g of water under vigorous stirring for one hour and then the liquid thickener was stored at 20°C.

The relevant data of the thus obtained liquid thickeners are reported in Table 4.

The viscosity was measured by means of a Brookfield viscometer RVT-DV-I after that each product had lost foam at 20°C.

Stability, expressed in months, is evaluated by visually checking the homogeneity of the sample.

Appearance is also evaluated visually.

The persistence of foam, expressed in days, is also reported in Table 4.

**Table 4**

| Ex. | Viscosity suppressing agent | Stability | Foam persistance | Appearance | Viscosity (mPa*s) |
|---|---|---|---|---|---|
| 22 | Sterol CC595 | > 6 | < 3 | transparent colourless | 4,000 |
| 23* | Sorbilene LH | > 6 | 10 | transparent yellowish | 181,400 |
| 24* | Emulson AG 7717/A | < 1 ** | >20 | transparent yellowish | / |
| 25* | BorchigenDFN | > 6 | 3 | slightly turbid yellowish | 18,,900 |
| 26* | Borchigen DFN + 5% of alkyne*** | > 6 | 3 | transparent yellowish | 9,450 |
| 27* | Tegodisperse 650 | < 1** | >20 | turbid colourless | / |

*Comparative example

**Separsted in two layers

***2,4,7,9-tetramethyl-5 decyne-4,7-diol (as in DE-A-4310702)

Comparing the data of Table 2 and Table 4, it is apparent that the same viscosity suppressing agents that are efficient with Polyurethane A (a medium effectiveness polyurethane associative thickener) are not so effective when a more pseudoplastic polyurethane (Polyurethane B) is used, while the ethoxylated glyceride maintains its very good performance even with Polyurethane B.

**Measurement of the thickening efficiency of the liquid thickeners of Examples 22, 25 and 26.**

2 g of the liquid thickener from Examples 22, 25 and 26 were each added to 98 g of a commercial acrylic emulsion NeoCryl XK-90 (from Avecia NeoResins), diluted to 36% of active content from 45%. The active content of thickener in each solution was 0.5% by weight.

The mixtures thus prepared were stirred for five minutes at 600 rpm. The thus obtained homogeneous dispersions were stored at 20 °C for 24 hours. The viscosity curves were then measured with the Physica UDS200 rheometer at 20 °C.

The results are set forth in Table 5, where the low shear and the high shear viscosity (in mPa*s) are reported.

**Table 5**

| Thickener from Example | Viscosity (0.01 s⁻¹) | Viscosity (10⁴ s⁻¹) |
|---|---|---|
| 22 | 1,300,000 | 76 |
| 25* | 778,000 | 53 |
| 26* | 1,000,000 | 59 |

*Comparative examples

The data of Table 4 and 5 clearly show that the ethoxylated glyceride of the invention, while being the most effective in lowering the viscosity of the concentrated composition, is also the best in maintaining the thickening efficiency of the polyurethane.

**Example 28-31. Preparation of liquid thickeners.**

Example 28

A reaction vessel, equipped with internal thermometer, stirrer and cooler, is filled, under nitrogen atmosphere and at room temperature with 214.4 g of a 4,000 average molecular weight polyethyleneglycol.

The polyethyleneglycol is melted, heated to 110 °C and dehydrated for 3 hours under vacuum; the temperature is then stabilised at 75 °C and the reactor content placed under a nitrogen blanket.

17.9 g of isophoronediisocyanate (IPDI) and 0.1 g of dibutyltin dilaurate as catalyst, are then added to the reactor under stirring.

The temperature is raised to 110 °C and the reaction is allowed to proceed until the titrimetric determination of the free NCO-groups gives the theoretical value.

Subsequently 17.6 g of 2-octyldodecanol are added.

After 1 hour of reaction, the polymer is checked to be NCO-negative according to the I.R. spectrum.

Finally the molten polymer is diluted with 200g of Sterol CC 595, 550 g of demineralized water to give a liquid thickener made of 25% polyurethane C/20% Sterol CC 595/55% water, having a Brookfield viscosity of 5620 mPa*s (S05; 50 rpm)

Examples 29-31.

Following the procedure described in Example 28, but using the reagents reported in Table 6, three more liquid thickeners were prepared.

Their Brookfield viscosity measured at 50 rpm is also reported in Table 6.

**Table 6**

| | **Ex. 28** | **Ex. 29** | **Ex. 30** | **Ex. 31** |
|---|---|---|---|---|
| polyether diol | PEG 4000 | PEG 4000 | PEG 4000 | PEG 4000 |
| (g) | (214.4) | (220.4) | (223.0) | (181.3) |
| capping agent (g) | OD³⁾ (17.6) | HD⁴⁾ (10.3) | HD⁴⁾ (10.5) | D⁵⁾ (5.2) |
| isocyanate (g) | IPDI (17.9) | HMDI¹⁾ (19.2) | IPDI (16.5) | IPDI (13.4) |
| ethoxylated | Sterol CC 595 | Sterol CC 595 | Sterol CC 595 | Sterol CC 595 |
| glyceride (g) | (200) | (250) | (120) | (50) |
| water (g) | (550) | (500) | (630) | (750) |
| thickener composition ²⁾ | 25/20/55 | 25/25/50 | 25/12/63 | 20/5/70 |
| Viscosity (mPa*s) | 5,620 | 10,200 | 8,200 | 10,280 |

1) hydrogenated 4,4'-diphenyl methane diisocyanate

2) polyurethane (%wt)/glyceride (%wt)/ water (%wt)

3) 2-octyldodecanol

4) 2-hexyldeanol

5) 1-decanol

**Thickening efficiency of the liquid thickeners of Examples 28-31.**

2 g of the liquid thickener from Examples 28, 29, 30 and 31 were each added to 98 g NeoCryl XK-90 (diluted to 36% of active content from 45%). The active content of thickener in each solution was always 0.5% by weight.

The mixtures thus prepared were stirred for five minutes at 600 rpm. The homogeneous dispersions thus obtained were stored at 20 °C for 24 hours. The viscosity curves were then measured with the Physica UDS200 rheometer at 20 °C.

The results are set forth in Table 7, where the low shear and the high shear viscosity (in mPa*s) are reported.

**Table 7**

| Example | Low shear viscosity (0.01 s-1) | High shear viscosity (10⁴ s-1) |
|---|---|---|
| 28 | 1,890,000 | 40 |
| 29 | 84,0000 | 47 |
| 30 | 72,000 | 55 |
| 31 | 1,557 | 147 |

The data listed in Table 7 clearly show that the viscosity suppressing agent of the present invention is really effective in lowering the viscosity of hydrophobically modified polyurethane associative thickeners of different rheology profile, from thickeners for low shear rate to thickeners for high shear rate.

**Thickening efficiency of the liquid thickener of**

**Example 28 and 30 in water.**

Here below we report in Table 8 the thickening in water of the liquid thickener of the present invention compared to three commercially available solvent free associative polyurethane thickeners of different rheology.

**Table 8**

| Thickener | Solids content in water (polymer + viscosity reducers) | Active content in water (polymer) | Viscosity mPa*s (10 s-1; 20 °C) |
|---|---|---|---|
| from Example 30 | 5.9% | 4% | 24,000 |
| from Example 28 | 7.2% | 4% | 175,000 |
| Acrysol RM 12W* | 4% | -- | 16 |
| Acrysol RM 12W* | 8% | -- | 72 |
| Borchigel L75N** | 8% | 4% | 6,000 |
| Tegoviscoplus 3060*** | 4% | -- | 738 |
| Tegoviscoplus 3060*** | 8% | -- | 750 |

* urethane associative thickener from Rohm and Haas

**urethane associative thickener for medium-high shear rate from Borchers

*** pseudoplastic urethane associative thickener from Tego

The liquid thickeners of the present invention are the only able to appreciatively thicken water and to give a transparent and colourless aqueous solution.

**Application examples**

In the following tables the active matter (active) percentage is reported when it differs from the dosed ingredient.

**Preparation of shower cream**

The ingredients listed in Table 9 are mixed.

The surfactants are first added to water, then the mixture is thickened with the liquid thickener and the other ingredients are added.

A soft and silky shower cream is obtained with physiological pH.

**Table 9**

| **INCI name** | **% active** | **quantity** |
|---|---|---|
| AQUA | | to 100 |
| SODIUM LAURETH SULFATE | 9.8 | 35 |
| COCAMIDOPROPYL BETAINE | 2.4 | 8 |
| PEG-13 SUNFLOWER GLYCERIDES | | 1.5 |
| POLYQUATERNIUM-7 | | 1.0 |
| GLYCERIN | | 3.0 |
| Liquid thickener from Example 28 | 2.5 | 10 |
| ALLANTOIN | | 0.5 |
| PERFUME | | QS |
| PRESERVATIVE | | QS |

**Preparation of a delicate shower gel.**

The ingredients listed in Table 10 are mixed.

**Table 10**

| **INCI Name** | **% active** | **quantity** |
|---|---|---|
| AQUA | | to 100 |
| SODIUM LAURETH SULFATE | | 35 |
| SODIUM COCOPOLYGLUCOSE | 10.5 | 35 |
| TARTRATE | | |
| PEG-7 GLYCERYL COCOATE | | 2.5 |
| GLYCERIN | | 2.0 |
| Liquid thickener from Example 28 | 2.5 | 10 |
| PERFUME | | QS |
| PRESERVATIVE | | QS |

A Newtonian shower gel is obtained, delicate on skin thanks to the presence of a detoxifying surfactant such as cocopolyglucose tartrate.

The addition of the liquid thickener does not alter the pH of the formulation.

**Preparation of an after-shampoo balm.**

The ingredients listed in Table 11 are mixed.

An after-shampoo balm is obtained with both the conditioning power of the wheat proteins and of guar and the soft touch of the liquid thickener of the invention.

The light filming properties of the thickener impart protection to the hair.

**Preparation of a liquid cleansing product**

The ingredients listed in Table 12 are mixed.

A simple and at the same time efficient formulation is obtained which is well suited to family needs.

The low content in LES, together with the presence of the betaine, imparts a certain delicacy to the product, which is highly transparent and colourless.

**Table 11**

| **INCI Name** | **active %** | **quantity** |
|---|---|---|
| AQUA | | to 100 |
| GUAR HYDROXYPROPYLTRIMONIUM | | 0.3 |
| CLORIDE | | |
| Liquid thickener from Example 28 | 2.5 | 10 |
| HYDROLIZED WHEAT PROTEIN | | 0.5 |
| CITRIC ACID | | 0.2 |
| PANTHENOL | | 0.5 |
| COCAMIDOPROPYL BETAINE | 1 | 3.3 |
| GLYCOL DISTEARATE (and) STEARETH-4 | | 3.0 |
| PERFUME | | QS |
| PRESERVATIVE | | QS |

**Table 12**

| **INCI Name** | **active %** | **quantity** |
|---|---|---|
| AQUA | | to 100 |
| SODIUM LAURETH SULFATE | 9.8 | 35 |
| COCAMIDOPROPYL BETAINE | 2.4 | 8 |
| Liquid thickener from Example 28 | 1.25 | 5 |
| PERFUME | | QS |
| PRESERVATIVE | | QS |

**Preparation of a balm-shampoo**

The ingredients listed in Table 13 are mixed.

A formulate is obtained combining the very good washing power of the surfactant mixture with the conditioning power of the wheat protein.

The thickener of the invention gives protection to the hair thanks to a delicate film. The formulate is highly transparent.

**Preparation of a delicate shampoo.**

The ingredients listed in Table 14 are mixed.

A shampoo is obtained that, thanks to the surfactant combination and to the detoxifying properties of tartrate against LES, possesses a highly delicate profile that allows daily use.

The formulate is light yellow and transparent.

**Table 13**

| **INCI Name** | **active %** | **quantity** |
|---|---|---|
| AQUA | | to 100 |
| SODIUM LAURETH SULFATE | 9.8 | 30 |
| SODIUM COCOPOLYGLUCOSE | 3 | 10 |
| TARTRATE | | |
| Liquid thickener from Example 28 | 1.25 | 5 |
| PEG-60 ALMOND GLYCERIDES | | 1.5 |
| HYDROLIZED WHEAT PROTEIN | | 1.5 |
| LACTIC ACID to pH 5.5 | | QS |
| PERFUME | | QS |
| PRESERVATIVE | | QS |

**Table 14**

| **INCI Name** | **active %** | **quantity** |
|---|---|---|
| AQUA | | to 100 |
| SODIUM LAURETH SULFATE | 5 | 17.85 |
| SODIUM COCOAMPHOACETATE | 5 | 16.67 |
| SODIUM COCOPOLYGLUCOSE TARTRATE | 5 | 16.67 |
| SODIUM COCOPOLYGLUCOSE SULFO- | 5 | 12.5 |
| SUCCINATE | | |
| Liquid thickener from Example 28 | 1.25 | 5 |
| HYDROLIZED WHEAT PROTEIN | | 1.5 |
| LACTIC ACID to pH 5.5 | | QS |
| PERFUME | | QS |
| PRESERVATIVE | | QS |

**Preparation of a shampoo for greasy hair.**

The ingredients listed in Table 15 are mixed.

A transparent light yellow gel is obtained.

The presence of cocopolyglucose contributes to the detoxifying action from LES offering at the same time high degreasing power.

**Preparation of a baby shampoo.**

The ingredients listed in Table 16 are mixed.

A shampoo is obtained which is suited to the delicate skin of babies.

The thickener of the invention fits well to the formulation having a very favourable toxicological profile.

**Table 15**

| **INCI Name** | **attivo %** | **quantity** |
|---|---|---|
| AQUA | | to 100 |
| SODIUM LAURETH SULFATE | 5.6 | 20 |
| COCAMIDOPROPYL BETAINE | 3 | 10 |
| SODIUM COCOPOLYGLUCOSE CITRATE | 3 | 10 |
| Liquid thickener from Example 28 | 2.5 | 10 |
| PERFUME | | QS |
| PRESERVATIVE | | QS |

**Table 16**

| **INCI Name** | **active %** | **quantity** |
|---|---|---|
| AQUA | | to 100 |
| SODIUM LAURETH SULFATE | 3.64 | 13 |
| DISODIUM COCOAMPHODIACETATE | 3.9 | 13 |
| SODIUM COCOPOLYGLUCOSE | 3.9 | 13 |
| TARTRATE | | |
| SODIUM COCOPOLYGLUCOSE SULFO- | 1.6 | 4 |
| SUCCINATE | | |
| Liquid thickener from Example 28 | 1.25 | 5 |
| PERFUME | | QS |
| PRESERVATIVE | | QS |

**Preparation of a protective milk.**

The ingredients listed in Table 17 are mixed.

Thanks to the combination of silicones, a dry touch protective milk is obtained.

The thickener of the invention stabilises and thickens the emulsion, also imparting a certain protection to skin, thanks to its low filming properties.

**Preparation of a purifying milk.**

The ingredients listed in Table 18 are mixed.

Thanks to salicylic acid, a purifying milk is obtained.

The thickener of the invention stabilises the formulation protecting salicylic acid from oxidation.

**Preparation of a hand cream.**

The ingredients listed in Table 19 are mixed.

A protective cream is obtained with silky aspect and very soft touch, due to the thickener of the invention.

The selection of oils makes the cream extremely light.

**Table 17**

| **INCI Name** | **active %** | **quantity** |
|---|---|---|
| (phase A): | | |
| AQUA | | to 100 |
| Liquid thickener from Example 28 | 2.5 | 10 |
| GLYCERIN | | 1.5 |
| PRESERVATIVE | | QS |
| (phase B): | | |
| CYCLOPENTASILOXANE | | 5.0 |
| DIMETHICONOL | | 5.0 |
| DIMETHICONE 200 cst | | 5.0 |
| C12-15 ALKYLBENZOATE | | 5.0 |
| PERFUME | | QS |

**Table 18**

| **INCI Name** | **active %** | **quantity** |
|---|---|---|
| (phase A): | | |
| AQUA | | to 100 |
| Liquid thickener from Example 28 | 2.5 | 10 |
| SALICYLIC ACID | | 0.5 |
| HAMAMELIS VIRGINIANA | | 0.5 |
| PRESERVATIVE | | QS |
| (phase B): | | |
| GLYCERYLSTEARATE (and) PEG-100 | | 8.0 |
| STEARATE | | |
| DIMETHICONE 200 cst | | 3.0 |
| PERFUME | | QS |

**Table 19**

| **INCI name** | **active %** | **quantity** |
|---|---|---|
| (phase A): | | |
| AQUA | | to 100 |
| Liquid thickener from Example 28 | 5 | 20 |
| GLYCERIN | | 5.0 |
| ALOE BARBADENSIS | | 0.5 |
| PRESERVATIVE | | QS |
| (phase B): | | |
| HYDROGENATED POLYDECENE | | 7.5 |
| DIMETHICONE 200 cst | | 2.5 |
| PERFUME | | QS |

Preparation of a hair gel balm.

The ingredients listed in Table 20 are mixed.

A non sticky, very transparent gel is obtained.

**Table 20**

| **INCI name** | **active %** | **quantity** |
|---|---|---|
| AQUA | | to 100 |
| Liquid thickener from Example 28 | 6.25 | 25 |
| HYDROLIZED WHEAT PROTEIN | | 2.0 |
| POLYSORBATE-20 | | 0.4 |
| PERFUME | | 0.1 |
| PRESERVATIVE | | QS |

Preparation of a protective after shave.

The ingredients listed in Table 21 are mixed with a dissolver.

**Table 21**

| **INCI name** | **active %** | **quantity** |
|---|---|---|
| AQUA | | to 100 |
| Liquid thickener from Example 28 | 2.5 | 10 |
| TRIDECETH-9 (e) PEG-40 HYDROGENATED | | 2.0 |
| CASTOR OIL | | |
| OCTYL OCTANOATE | | 3.0 |
| BISABOLOL | | 0.5 |
| SACCHARIDE ISOMERATE | | 1.0 |
| MENTHOL | | 0.1 |
| PRESERVATIVE | | QS |
| PERFUME | | QS |

The thickener gives viscosity to the product and stabilises the less water soluble components. A product is obtained that, spread on skin, forms a light protective film.

**Preparation of a detergent gel for sanitary ware and hard surfaces.**

The ingredients listed in Table 22 are mixed.

A completely transparent product is obtained with Brookfield viscosity of 3,000 mPa*s, at 20 rpm and 25°C.

When contacted with scale, the product forms an easily removable consistent mousse.

**Table 22**

| **Ingredient** | **active %** | **quantity** |
|---|---|---|
| Water | | to 100 |
| Liquid thickener from Example 28 | 2.5 | 10 |
| Cocopolyglucoside citrate, disodium salt | 2.1 | 7 |
| Citric acid (to pH 2.5 - 3.0) | 1.4 | 7 |

## Claims

1. Liquid thickeners for aqueous systems containing: a) from 5 to 40% wt of ethoxylated mono- and/or di-glycerides of carboxylic acids having from6 to 18 carbon atoms; b) from 15 to 40% wt of a polyurethane associative thickener, obtained from the reaction of at least one organic polyisocyanate, at least one water soluble polyether diol and at least one hydrophobic capping agent containing from 10 to 24 carbon atoms; c) from 20 to 80% wt of water, devoid of organic solvents, volatile organic compounds and other viscosity suppressing agents.

2. Liquid thickeners according to claim 1. wherein the mono and/or di-glycerides are ethoxylated with from 4 to 12 moles of ethylene oxide.

3. Liquid thickeners according to claim 2. wherein the associative polyurethane thickener is obtained from the reaction of at least one organic polyisocyanate, at least one water soluble polyether diol and at least one hydrophobic capping agent containing from 12 to 24 carbon atoms.

4. Liquid thickeners according to claim 3., wherein the ethoxylated mono- and/or di-glycerides are glycerides of saturated fatty acids having from 8 to 14 carbon atoms, ethoxylated with about 6 to 7 moles of ethylene oxide.

5. Liquid thickeners according to claim 4. containing: a) from 5 to 30% wt of mono and/or di-glycerides of caprylic and capric acids ethoxylated with about 6 moles of ethylene oxide; b) from 15 to 30% wt of an associative polyurethane obtained from the reaction of polyethylene glycol, 4,4'-dicyclohexylmethane diisocyanate and/or 1-isocyanatomethyl-5-isocyanato-1,3,3-trimethylcyclohexane and C₁₆-C₂₄ branched monohydric alcohol, preferably C₁₆ or C₂₀ Guerbet alcohol, and having a molecular weight of about 6,000 to 20,000; c) from 40 to 80% wt of water.

6. Process for the preparation of liquid thickeners, comprising the following steps a) reacting at least one organic polyisocyanate, at least a water soluble polyether diol and at least a hydrophobic capping agent containing from 10 to 24 carbon atoms, to obtain an associative polyurethane; b) mixing the polyurethane with from 0.1 to 2.7 parts by weight of ethoxylated mono and/or di-glycerides of carboxylic acids having from 6 to 18 carbon atoms and with from 0.5 to 5.3 parts by weight of water.

7. A process for the preparation of liquid thickener according to claim 6., wherein the polyurethane of steps a) and b) is in molten form.

8. A cosmetic composition comprising a liquid thickener of any of claims from 1. to 5.

9. A latex paint comprising a liquid thickener of any of claims from 1. to 5.

10. A household cleaner composition comprising a liquid thickener of any of claims from 1. to 5.

11. Use of liquid thickeners of claims from 1. to 5. for the preparation of cosmetics; latex paints; water-based paper coatings; household cleaners.

## Patentansprüche

1. Flüssigkeitsverdicker für wässrige Systeme, folgendes enthaltend: a) zwischen 5 und 40% Gewichtsanteil ethoxylierte Mono- und/oder Diglyceride von Carboxylsäuren, zwischen 6 und 18 Kohlenstoffatome enthaltend; b) zwischen 15 und 40% Gewichtsanteile eines assoziativen Polyurethan-Verdickers, der aus der Reaktion von mindestens einem organischen Polyisocyanat, von mindestens einem wasserlöslichen Polyätherdiol und von mindestens einem hydrophoben Verkappungsmittel gewonnen wird, zwischen 10 und 24 Kohlenstoffatome enthaltend; c) zwischen 20 und 80% Gewichtsanteile Wasser, das frei von organischen Lösungsmitteln, flüchtigen organischen Gemischen und anderen die Viskosität verringernden Mitteln ist.

2. Flüssigkeitsverdicker gemäß Patentanspruch 1, worin die Mono- und/oder Diglyceride mit 4 bis 12 Mol Äthylenoxid ethoxyliert werden.

3. Flüssigkeitsverdicker gemäß Patentanspruch 2, worin der Polyurethan enthaltende Verdicker aus der Reaktion von mindestens einem organischen Polyisocyanat, von mindestens einem wässerlöslichen Polyätherdiol und von mindestens einem hydrophoben Verkappungsmittel gewonnen wird, zwischen 12 und 24 Kohlenstoffatome enthaltend.

4. Flüssigkeitsverdicker gemäß Patentanspruch 3, worin die ethoxylierten Mono- und/oder Diglyceride Glyceride von gesättigten Fettsäuren mit 8 bis 14 Kohlenstoffatome sind, die mit ungefähr 6 bis 7 Mol Äthylenoxid ethoxyliert wurden

5. Flüssigkeitsverdicker gemäß Patentanspruch 4, folgendes enthaltend: zwischen 5 und 30% Gewichtsanteile Mono- und/oder Diglyceride von Capryl- und Caprinsäuren, die mit ungefähr 6 Mol Äthylenoxid ethoxyliert wurden; b) zwischen 15 bis 30% Gewichtsanteile eines assoziativen Polyurethans, das aus der Reaktion eines Polyäthylenglicols, 4,4'-Dicyclohexylmethan Diisocyanat und/oder 1-Isocyanatomethyl-5-isocyanato-1,3,3-trimethylcyclohexan und einem C₁₃-C₂₄ verzweigten monohydrischen Alkohol, vorzugsweise C₁₆ oder C₂₀ Guerbet-Alkohol, gewonnen wird und ein Molekulargewicht von ungefähr 6,000 bis 20,000 aufweist; c) zwischen 40 und 80% Gewichtsanteile Wasser.

6. Prozess zur Zubereitung von Flüssigkeitsverdickern, der folgende Schritte beinhaltet: a) Reaktion von mindestens einem organischen Polyisocyanat, von mindestens einem wasserlöslichen Polyätherdiol und von mindestens einem hydrophoben Verkappungsmittel, zwischen 10 und 24 Kohlenstoffatome enthaltend, um ein assoziatives Polyurethan zu erhalten; b) Mischen des Polyurethans mit 0.1 bis 2.7 Gewichtsanteilen ethoxylierter Mono- und/oder Diglyceride von Carboxylsäuren, zwischen 6 und 18 Kohlenstoffatome enthaltend, und mit 0.5 bis 5.3 Gewichtsanteilen Wasser.

7. Prozess zur Zubereitung eines Flüssigkeitsverdickers gemäß Patenanspruch 6, worin das Polyurethan der Schritte a) und b) in geschmolzener Form vorliegt.

8. Eine kosmetische Zusammensetzung, die einen der in den Patentansprüchen 1. bis 5. aufgeführten Flüssigkeitsverdicker enthält.

9. Eine Latexfarbe, die einen der in den Patentansprüchen 1. bis 5. aufgeführten Flüssigkeitsverdicker enthält.

10. Eine Haushaltsreinigerzusammensetzung, die einen der in den Patentansprüchen 1. bis 5. aufgeführten Flüssigkeitsverdicker enthält.

11. Verwendung eines Flüssigkeitsverdickers der Patenansprüche 1. bis 5. zur Zubereitung von Kosmetika; Latexfarben; wasserbasierenden Papierbeschichtungen; Haushaltsreinigern.

## Revendications

1. Épaississants liquides destinés à des systèmes aqueux et contenant : a) de 5 à 40 % en poids de mono- et/ou diglycérides éthoxylés d'acides carboxyliques ayant de 6 à 18 atomes de carbone ; b) de 15 à 40 % en poids d'un épaississant associatif polyuréthanne, obtenu en faisant réagir au moins un polyisocyanate organique, au moins un polyéther diol hydrosoluble et au moins un agent de coiffage hydrophobe contenant de 10 à 24 atomes de carbone ; c) de 20 à 80 % en poids d'eau, lesdits épaississant étant dépourvus de solvants organiques, de composés organiques volatils et autres agents supprimant la viscosité.

2. Épaississants liquides selon la revendication 1, lesdits mono- et/ou diglycérides étant éthoxylés avec 4 à 12 moles d'oxyde d'éthylène.

3. Épaississants liquides selon la revendication 2, ledit épaississant associatif polyuréthanne étant obtenu en faisant réagir au moins un polyisocyanate organique avec, au moins un polyéther diol hydrosoluble et au moins un agent de coiffage hydrophobe contenant de 12 à 24 atomes de carbone.

4. Épaississants liquides selon la revendication 3, lesdits mono- et/ou diglycérides éthoxylés étant des glycérides d'acides gras saturés ayant de 8 à 14 atomes de carbone, éthoxylés avec environ 6 à 7 moles d'oxyde d'éthylène.

5. Épaississants liquides selon la revendication 4, contenant ; a) de 5 à 30 % en poids de mono- et/ou diglycérides d'acides caprylique et caprique éthoxylés avec environ 6 moles d'oxyde d'éthyléne ; b) de 15 à 30 % en poids d'un polyuréthanne associatif obtenu en faisant réagir du polyéthylène glycol, du 4,4'-dicyclohexylméthane diisocyanate et/ou du 1-isocyanatométhyl-5-isocyanato-1,3,3-triméthylcyclohexane et un alcool monohydrique ramifié en C₁₆ à C₂₄, de préférence un alcool de Guerbet en C₁₆ ou C₂₀, et ayant une masse moléculaire d'environ 6000 à 20 000 ; c) de 40 à 80 % en poids d'eau.

6. Procédé de préparation d'épaississants liquides comprenant les étapes, consistant à : a) faire réagir au moins un polyisocyanate organique avec au moins un polyéther diol hydrosoluble et au moins un agent de coiffage hydrophobe contenant de 10 à 24 atomes de carbone, pour obtenir un polyuréthanne associatif ; b) mélanger ledit polyuréthanne avec de 0,1 à 2,7 parties en poids de mono- et/ou diglycérides éthoxylés d'acides carboxyliques ayant de 6 à 18 atomes de carbone, ainsi qu'avec de 0,5 à 5,3 parties en poids d'eau.

7. Procédé de préparation d'épaississant liquide selon la revendication 6, le polyuréthanne des étapes a) et b) étant sous forme fondue.

8. Composition cosmétique comprenant un épaississant liquide selon l'une quelconque des revendications 1 à 5.

9. Peinture au latex comprenant un épaississant liquide selon l'une quelconque des revendications 1 à 5.

10. Composition de nettoyage pour la maison comprenant un épaississant liquide selon l'une quelconque des revendications 1 à 5.

11. Utilisation d'épaississants liquides des revendications 1 à 5 pour préparer des cosmétiques, des peintures au latex, des revêtements pour papier à base d'eau et des produits de nettoyage pour la maison.
